Europäisches Patentamt

European Patent Office

Office européen des brevets

(19)

(11) Veröffentlichungsnummer: **0 271 772**
**A2**

(12) # EUROPÄISCHE PATENTANMELDUNG

(21) Anmeldenummer: 87117831.5

(22) Anmeldetag: 02.12.87

(51) Int. Cl.⁴: **C08L 63/00** , C08K 13/04 , C08K 3/36 , H01L 23/30 , C08G 59/50

(30) Priorität: 15.12.86 DE 3642782

(43) Veröffentlichungstag der Anmeldung:
22.06.88 Patentblatt 88/25

(84) Benannte Vertragsstaaten:
AT DE FR GB NL

(71) Anmelder: Siemens Aktiengesellschaft Berlin und München
Wittelsbacherplatz 2
D-8000 München 2(DE)

(72) Erfinder: Kleeberg, Wolfgang, Dr.Dipl.-Chem.
Hessenstrasse 7
D-8520 Erlangen(DE)
Erfinder: Hacker, Heinz, Dr. Dipl.-Chem.
Kaiserslauterer Strasse 9
D-8500 Nürnberg(DE)
Erfinder: Laupenmühlen, Heinz Klaus
Dr. Georg-Dassler-Strasse 20
D-8551 Hemhofen(DE)
Erfinder: Huber, Jürgen
Am Heiligenholz 6
D-8520 Erlangen(DE)
Erfinder: Wilhelm, Dieter, Dr. Dipl.-Chem.
Schubertstrasse 5
D-8550 Forchheim(DE)

(54) Epoxidharz-Formmassen.

(57) Formmassen zur Umhüllung von Halbleiterbauelementen auf der Basis von füllstoffhaltigen Polyepoxidharzen und Polyaminen als Härter sind nicht lagerstabil. Die neuen Epoxidharz-Formmassen sollen einerseits lagerstabil sein, wobei andererseits aber gewährleistet sein soll, daß die daraus herzustellenden Formstoffe den an diese Werkstoffe gestellten Anforderungen genügen.

Die Formmassen nach der Erfindung enthalten als Härter ein 1.3.5-Tris(3-amino-4-alkylphenyl)-2.4.6-trioxo-hexahydrotriazin mit einem $C_1$-bis $C_4$-Alkylrest.

Hüllmasssen für Halbleiterbauekemente

EP 0 271 772 A2

## Epoxidharz-Formmassen

Die Erfindung betrifft Formmassen zur Umhüllung von Halbleiter-bauelementen, bestehend aus einem aromatischen und/oder heterocyclischen Polyepoxidharz und einem aromatischen Polyamin als Härter sowie pulverförmigen mineralischen Füllstoffen, gegebenenfalls im Gemisch mit anorganischen Kurzfasern, sowie aus derartigen Formmassen hergestellte Formstoffe.

Vernetzbare, d.h. härtbare Harzsysteme sind von besonderer Bedeutung für die Isolierstofftechnik, insbesondere als Harzmatrizes für Preßmassen. Derartige Harzsysteme sind beispielsweise Epoxidharz-Formmassen, insbesondere in Form der Reaktionsharz-Formmassen Typ 870 und Typ 871 (siehe dazu: H. Saechtling "Kunststoff-Taschenbuch", 20. Ausgabe, 1977, Seite 416 bzw. Tafel 85), d.h. Epoxidharz-Preßmassen (nach DIN 16912) mit anorganischem körnigem bzw. anorganischem kurzfaserigem Füllstoff.

Epoxidharz-Formmassen, insbesondere solche mit aminischen Härtern auf der Basis aromatischer Amine, wie 4.4'-Diaminodiphenylsulfon (siehe beispielsweise DE-OS 32 10 746), liefern im vernetzten Zustand zwar hochwertige Formstoffe, sie sind aber nur begrenzt lagerstabil, d.h. sie erlauben - aufgrund der stetig fortschreitenden Reaktion zwischen Epoxid und Amin, welche selbst bei niedrigen Temperaturen rasch voranschreitet, - keine Einstellung eines eigenschaftsstationären Zustandes. Die stetig fortschreitende chemische Reaktion zwischen dem Oxiran und dem Aminwasserstoff bedingt ferner eine entsprechende Änderung der Verarbeitungsparameter und beeinflußt unter Umständen auch die Qualität der aus den Formmassen hergestellten Formstoffe.

Aus Polyepoxidharzen, d.h. Polyglycidylverbindungen, und Polyaminen mit Isocyanuratstrukturelementen, wie sie aus der DE-PS 27 43 680 bekannt sind, lassen sich Formstoffe mit hoher Wärmeformbeständigkeit und hoher Dimensionsstabilität herstellen. Das Problem der eingeschränkten Verarbeitbarkeit zeigt sich aber auch bei derartigen Formmassen deutlich.

Aufgabe der Erfindung ist es, Formmassen der eingangs genannten Art auf der Basis von Polyepoxidharzen und einem Polyamin als Härter derart auszugestalten, daß sie lagerstabil sind, wobei andererseits aber gewährleistet sein muß, daß die aus diesen Formmassen herzustellenden Formstoffe die von diesen Werkstoffen geforderten Eigenschaften erfüllen.

Dies wird erfindungsgemäß dadurch erreicht, daß der Härter ein 1.3.5-Tris(3-amino-4-alkylphenyl)-2.4.6-trioxo-hexahydrotriazin mit einem $C_1$- bis $C_4$-Alkylrest ist.

Gegenstand der Erfindung sind somit Formmassen zur Umhüllung von Halbleiterbauelementen auf der Basis von füllstoffhaltigen Polyepoxidharzen, welche als Härter ein Polyamin in Form eines Isocyanursäurederivates der vorstehend genannten Art enthalten. Gegenstand der Erfindung sind ferner aus derartigen Formmassen hergestellte Epoxidharz-Formstoffe.

Die erfindungsgemäßen Epoxidharz-Formmassen weisen eine hohe Lagerstabilität auf. Bei diesen Formmassen wird die Polyadditionsreaktion bei Raumtemperatur nämlich so weit verzögert, daß über einen Zeitraum von mindestens 12 Wochen (bei Raumtemperatur) eine einwandfreie Verarbeitbarkeit erhalten bleibt; bei Temperaturen oberhalb 150°C erfolgt dagegen eine rasche Vernetzung. Diese Formmassen zeichnen sich ferner durch einen geringen Reaktionsschwund (< 6 %) aus.

Die aus den Formmassen hergestellten erfindungsgemäßen Form stoffe weisen neben einer hohen Glasübergangstemperatur ($T_g$ > 200°C) einen niedrigen linearen Wärmeausdehnungskoeffizienten auf, und zwar im Temperaturbereich von Raumtemperatur bis ca. 210°C; der Wärmeausdehnungskoeffizient liegt - abhängig von Füllstoffart und Füllstoffgehalt - im Bereich von 15 bis 25 $\times$ 10$^6$ K$^1$ . Wegen des fehlenden $\alpha$-Sprungs, d.h. der Änderung des linearen Wärmeausdehnungskoeffizienten im Glasübergangsbereich, werden im Anwendungsbereich außerdem verringerte Spannungen auf das Gesamtsystem ausgeübt. Die erfindungsgemäßen Formstoffe sind außerdem selbstverlöschend: Mit einer mittleren Brenndauer von 5 Sekunden (ohne Halogenzusatz) gelten sie nach IEC 249-1 als schwer brennbar. Ferner zeigen diese Formstoffe eine gute Haftfestigkeit auf Metallen, was bei der Umhüllung von Halbleiterbauelementen sehr wichtig ist, und darüber hinaus wird dabei die Dichtigkeit der Beinchen ("pins") gegenüber eindringender Feuchtigkeit gewährleistet.

Die in den erfindungsgemäßen Formmassen als Härter eingesetzten Verbindungen sind aromatische Aminogruppen aufweisende primäre Polyamine. Derartige Polyamine können beispielsweise durch Hydrolyse entsprechender Verbindungen mit freien Isocyanatgruppen hergestellt werden. Ein Verfahren dieser Art ist beispielsweise aus der DE-OS 32 27 219 bekannt. Die nach dem bekannten Verfahren hergestellten Polyamine dienen zur Herstellung von Polyurethanen, Polyurethankunststoffen und Polyurethanschaumstoffen. Als eine unter mehreren weiteren Verwendungsmöglichkeiten der Polyamine ist in der DE-OS 32 27 219 zwar "Härter für Epoxid-und Phenolharze" angegeben, es ist aber durchaus überraschend und konnte keineswegs vorhergesehen werden, daß aus der großen Zahl der dort aufgeführten Verbindungen gerade ein spezieller Polyamintyp, nämlich 1.3.5-Tris(3-amino-4-alkylphenyl)-2.4.6-trioxo-hexahydrotria-zin, als Härter in füllstoffhaltigen Epoxidharz-Formmassen zur Umhüllung von Halbleiterbauelementen eingesetzt werden kann, die eine hohe Lagerstabilität aufweisen und zu Epoxidharz-Formstoffen mit einem hohen Eigenschaftsniveau führen.

Der in den erfindungsgemäßen Formmassen eingesetzte Härter wird aus 2.4-Diisocyanato-alkylbenzolen, wie 2.4-Diisocyanatotoluol, hergestellt. Die Diisocyanato-alkylbenzole (Alkyl = $CH_3$, $C_2H_5$, $C_3H_7$ oder $C_4H_9$) werden dabei mittels geeigneter Katalysatoren trimerisiert und in 1.3.5-Tris(3-isocyanato-4-alkylphenyl)-2.4.6-trioxo-hexahydrotriazine übergeführt. Durch Hydrolyse der Isocyanatgruppen werden daraus dann die Polyamine gebildet.

Wenn in dem bei der Trimerisierung erhaltenen Reaktionsgemisch nicht umgesetztes Ausgangsmaterial, d.h. monomeres Diisocyanato-alkylbenzol, vorhanden ist, werden bei der Hydrolyse als Nebenprodukt aromatische Polyamine gebildet. Derartige Polyamine können in den erfindungsgemäßen Formmassen - neben dem eigentlichen Härter - als additive Härterkomponente eingesetzt werden.

Bei der Hydrolyse des isocyanatgruppenhaltigen Trimerisierungsproduktes kann es auch zu einer Reaktion zwischen Isocyanatgruppen und Aminogruppen kommen. Dabei werden, als Nebenprodukt der Hydrolysereaktion, heterocyclische Polyamine mit Harnstoffgruppierungen erhalten. Derartige Polyamine können in den erfindungsgemäßen Formmassen ebenfalls als additive Härterkomponente eingesetzt werden, d.h. im Gemisch mit dem eigentlichen Härter.

In den erfindungsgemäßen Formmassen finden als Härter somit bevorzugt Hydrolyseprodukte von trimerisierten Diisocyanato-alkylbenzolen Verwendung, d.h. Härtergemische. Bei Härtergemischen dieser Art beträgt der Anteil des eigentlichen Härters, d.h. 1.3.5-Tris(3-amino-4-alkylphenyl)-2.4.6-trioxo-hexahydrotria-zin, im Gemisch vorteilhaft wenigstens 50 Gew.-%, vorzugsweise zwischen 80 und 95 Gew.-%. Das Verhältnis zwischen eingesetzter Epoxid-Funktion und eingesetzter Aminwasserstoff-Funktion (NH) beträgt bei den erfindungsgemäßen Formmassen im übrigen vorteilhaft 0,9:1 bis 1,1:1, vorzugsweise etwa 1:1.

Neben dem eigentlichen Härter bzw. neben Härtergemischen der vorstehend genannten Art können in den erfindungsgemäßen Formmassen auch aromatische Polyamine anderer Art, wie 4.4'-Diaminodiphenylmethan und 4.4'-Diaminodiphenylsulfon, und/oder andere heterocyclische Polyamine eingesetzt werden. Der Anteil derartiger Polyamine im Härtergemisch beträgt maximal 5 Gew.-%.

Geeignete Polyepoxide, d.h. Polyglycidylverbindungen mit wenigstens zwei Epoxidgruppen pro Molekül, sind epoxidierte Novolake, Polyglycidylether auf Bisphenol A-, Bisphenol F-und Bisphenol B-Basis sowie epoxidierte Hydantoine und Triglycidylisocyanurat; die Epoxide können auch im Gemisch vorliegen. Bevorzugt ist das Polyepoxidharz ein epoxidierter Novolak mit einer Epoxidzahl von 0,3 bis 0,6 und einem Gesamtgehalt an hydrolysierbarem Halogen < 0,6 Gew.-%, vorzugsweise < 0,1 Gew.-%. Vorteilhaft kann der epoxidierte Novolak zusammen mit Triglycidylisocyanurat eingesetzt werden. Ferner kann das Polyepoxidharz vorteilhaft ein Gemisch aus einem epoxidierten Novolak und einem kernhalogenierten Bisphenol-bisglycidylether und/oder einem kernhalogenierten epoxidierten Novolak sein, wobei der Gesamtgehalt des Gemisches an kerngebundenem Halogen maximal 4 Gew.-% beträgt. Aus Formmassen der letztgenannten Art, d.h. halogenhaltigen Formmassen, resultieren Epoxidharz-Formstoffe mit weiter verbesserter Schwerentflammbarkeit. Derartige Formstoffe erreichen nämlich eine Schwerentflammbarkeit der Stufe UL 94 V-0.

Als Füllstoffe dienen in den erfindungsgemäßen Formmassen pulverförmige mineralische Stoffe. Diese mineralischen Füllstoffe können auch im Gemisch mit anorganischen Kurzfasern in Form von Textilglas-Kurzfasern zum Einsatz gelangen. Textilglas-Kurzfasern weisen - entsprechend DIN 61850 - eine Faserlänge unter 1 mm auf; die mittlere Faserlänge beträgt vorzugsweise etwa 0,2 mm. Als mineralische Füllstoffe, die mit oder ohne Haftvermittler verwendet werden können, können Carbonate, wie Dolomit - (beispielsweise in der Form von Microdol®), sowie Aluminiumoxide und -oxidhydrate eingesetzt werden. Bevorzugt dienen als Füllstoffe α-strahlungsarme Mehle aus Quarz oder Quarzgut, insbesondere aus α-strahlungsarmen Rohstoffen, wie Bergkristall, oder Siliciumdioxid ($SiO_2$) mit geringer α-Aktivität, wie es beispielsweise aus der DE-OS 33 23 844 bekannt ist. Derartiges Siliciumdioxid ist frei von Verunreinigungen, insbesondere von den für die α-Emission verantwortlichen Elementen Thorium und Uran; es weist deshalb Emissionsraten < 0,01 α-Teilchen/cm$^2$.h auf.

Der Füllstoffanteil in den erfindungsgemäßen Formmassen beträgt im allgemeinen 50 bis 85 Gew.-%; vorzugsweise beträgt dieser Anteil 70 bis 80 Gew.-%. Enthält der Füllstoff, neben pulverförmigen mineralischen Stoffen, auch Textilglas-Kurzfasern, so beträgt der Faseranteil im Füllstoffgemisch 3 bis 40 Gew.-%. Als Füllstoffe kommen - allein oder im Gemisch mit anderen mineralischen Füllstoffen - auch faserige Füllstoffe auf mineralischer Basis in Betracht, beispielsweise Calciumsilicate, wie Wollastonit.

Die Aufbereitung der erfindungsgemäßen Formmassen kann in der Weise erfolgen, daß Polyepoxidharz, Polyamin (Härter) und Füllstoff auf geeigneten Mischeinrichtungen, wie einem Walzenstuhl, direkt miteinander vermischt werden (sogenannte trockene Aufbereitung). Es kann jedoch auch in der Weise vorgegangen werden, daß die Füllstoffe in eine Lösung von Polyepoxidharz und Härter in einem geeigneten organischen Lösungsmittel eingebracht werden (sogenannte nasse Aufbereitung). Derartige Lösungsmittel sind beispielsweise Aceton, Ethylacetat, Methylethylketon und Methylglykol (2-Methoxyethanol), wobei insbesondere ketonische Lösungsmittel zum Einsatz gelangen, vorzugsweise Aceton. Die Lösungen weisen dabei einen Gehalt von 40 bis 60 Gew.-%, vorzugsweise ca. 50 Gew.-%, an Polyepoxidharz und Härter auf. Nach dem Einbringen der Füllstoffe und dem Vermischen wird von den Lösungen das Lösungsmittel im Vakuum entfernt, beispielsweise bei einem Druck von 1 mbar und einer Temperatur von 60°C.

Die erfindungsgemäßen Formmassen werden im allgemeinen nach üblichen Transferpreßverfahren verarbeitet, d.h. sie werden unter erhöhtem Druck und erhöhter Temperatur verpreßt. Bei Temperaturen oberhalb 150°C werden die erfindungsgemäßen Formmassen dann in einer rasch verlaufenden Vernetzungsreaktion in die entsprechenden Formstoffe übergeführt.

Anhand von Ausführungsbeispielen soll die Erfindung noch näher erläutert werden.

Beispiel 1

Herstellung von 1.3.5-Tris(3-isocyanato-4-methylphenyl)-2.4.6-trioxo-hexahydrotriazin

500 g 2.4-Diisocyanatotoluol (NCO-Gehalt: 48,2 %) werden mit Natriumbenzoat, gelöst in Dimethylformamid, trimerisiert (siehe dazu US-PS 2 801 244). Nach etwa 1 h wird die Reaktion bei einem NCO-Wert von 24,8 % durch Zugabe von p-Toluolsulfonsäure gestoppt.

Beispiel 2

Herstellung von 1.3.5-Tris(3-amino-4-methylphenyl)-2.4-6-trioxo-hexahydrotriazin

Die Hydrolyse von Isocyanatverbindungen zu den entsprechenden Aminen ist in der Literatur hinreichend beschrieben. Beispiele für die Hydrolyse mit Hilfe von basischen und sauren Katalysatoren finden sich in FR-PS 14 15 317, DE-AS 11 55 907, DE-OS 29 48 419, DE-OS 30 39 600, DE-OS 31 12 118, DE-OS 32 44 912 und DE-OS 32 44 913. Eine andere Möglichkeit der Aminsynthese beruht auf der thermischen Spaltung von Addi tionsprodukten aus Isocyanaten und Alkohol, wie sie beispielsweise in DE-AS 12 70 046 und DE-OS 30 35 639 beschrieben ist. Die Hydrolyse von Isocyanatverbindungen verläuft besonders vorteilhaft mit konzentrierter Schwefelsäure (entsprechend DE-PS 27 43 680) oder in hochsiedenden Lösungsmitteln (entsprechend DE-OS 32 27 219).

Bei der Hydrolyse von 1.3.5-Tris(3-isocyanato-4-methylphenyl)-2.4.6-trioxo-hexahydrotriazin entsprechend DE-OS 32 27 219 wird einer heißen Mischung aus Dimethylacetamid, Wasser und Florisil als Katalysator (Florisil®, ein Produkt der Fa. Floridin Corp., besteht im wesentlichen aus Magnesiumsilicat) eine Lösung des nach Beispiel 1 hergestellten Trimerisats in Dimethylacetamid zugesetzt. Nach der Aufarbeitung wird ein Gemisch aus 1.3.5-Tris(3-amino-4-methylphenyl)-2.4.6-trioxo-hexahydrotriazin und heterocyclischen Polyaminen mit einem $NH_2$-Gehalt von 6,8 Gew.-% erhalten.

Beispiel 3

Herstellung von Formmassen

a) Aufbereitung in einem Lösungsmittel (nasse Aufbereitung) 450 Gewichtsteile eines epoxidierten Novolaks mit einem Epoxidwert von 0,57 werden in 450 Gewichtsteilen Aceton gelöst. Zu dieser Lösung wird eine Lösung von 300 Gewichtsteilen des nach Beispiel 2 hergestellten Härtergemisches ($NH_2$-Gehalt: 6,8 Gew.-%) in 300 Gewichtsteilen Aceton gegeben. Die auf diese Weise erhaltene 50 %ige Harz/Härter-Lösung wird mit 1630 Gewichtsteilen Quarzmehl, beispielsweise in Form von Silbond® der Fa. Quarzwerke GmbH, versetzt und gemischt. Das Gemisch wird dann auf eine Aluminiumfolie ausgegossen und das Lösungsmittel in einem Vakuumtrockenschrank entfernt (Druck: 1 mbar; Temperatur: 60°C; Dauer: 30 min). Die dabei erhaltene rieselfähige Masse ist bei 5°C 6 Monate und bei Raumtemperatur 3 Monate lagerstabil.

b) Trockene Aufbereitung 600 Gewichtsteile eines epoxidierten Novolaks mit einem Epoxidwert von 0,57 werden zusammen mit 400 Gewichtsteilen des nach Beispiel 2 hergestellten Härtergemisches ($NH_2$-Gehalt: 6,8 Gew.-%) und 2170 Gewichtsteilen Quarzmehl (beispielsweise in Form von Silbond®) auf einem Walzenstuhl 30 min lang durchmischt (Walzentemperatur: 60°C). Dabei wird eine rieselfähige Masse erhalten, die bei Raumtemperatur mehr als 3 Monate und bei 5°C mehr als 6 Monate lagerstabil ist.

Beispiel 4

Herstellung von Formstoffen in Form von Prüfkörpern

Lagerstabile Formmassen entsprechend Beispiel 3 werden nach üblichen Transferpreßverfahren zu Prüfkörpern verarbeitet (Preßtemperatur: 175°C; Preßdruck: 100 bar; Preßdauer: 5 min). Die Messung der thermisch-mechanischen Eigenschaften an den Prüfkörpern, die nach üblichen DIN-Methoden erfolgte, erbrachte folgende Ergebnisse:

- Glasübergangstemperatur $T_g$ : ohne Tempern : 210°C
  (nach DIN 53445)         2 h bei 190°C getempert: 220°C
                           2 h bei 210°C getempert: 233°C
                           2 h bei 220°C getempert: 240°C

- Biegespannung          :     Nasse Aufbereitung
  (nach DIN 53452)        ungetempert            getempert[*]
                          $168 \pm 17 \frac{N}{mm^2}$      $134 \pm 30 \frac{N}{mm^2}$

                                Trockene Aufbereitung
                                      getempert[*]
                                $162 \pm 18 \frac{N}{mm^2}$

                          [*] bis 220°C


- Schlagzähigkeit        :       Nasse Aufbereitung
  (nach DIN 53453)        ungetempert            getempert[*]
                          $5,7 \pm 1,4 \frac{Nmm}{mm^2}$      $6,2 \pm 1,0 \frac{Nmm}{mm^2}$

                                Trockene Aufbereitung
                                      getempert[*]
                                $6,1 \pm 1,1 \frac{Nmm}{mm^2}$

                          [*] bis 220°C

**Ansprüche**

1. Formmassen zur Umhüllung von Halbleiterbauelementen, bestehend aus einem aromatischen und/oder heterocyclischen Polyepoxidharz und einem aromatischen Polyamin als Härter sowie pulverförmigen mineralischen Füllstoffen, gegebenenfalls im Gemisch mit anorganischen Kurzfasern, **dadurch gekennzeichnet,** daß der Härter 1.3.5-Tris(3-amino-4-alkylphenyl)-2.4.6-trioxo-hexahydrotriazin mit einem $C_1$-bis $C_4$-Alkylrest ist.

2. Formmassen nach Anspruch 1, **dadurch gekennzeichnet,** daß der Härter im Gemisch mit weiteren aromatischen und/oder heterocyclischen Polyaminen vorliegt.

3. Formmassen nach Anspruch 2, **dadurch gekennzeichnet,** daß der Anteil an 1.3.5-Tris(3-amino-4-alkylphenyl)-2.4.6-trioxo-hexahydrotriazin im Härtergemisch wenigstens 50 Gew.-%, vorzugsweise zwischen 80 und 95 Gew.-%, beträgt.

4. Formmassen nach einem der Ansprüche 1 bis 3, **dadurch gekennzeichnet,** daß das Verhältnis zwischen Epoxid-und Aminwasserstoff-Funktion 0,9:1 bis 1,1:1, vorzugsweise etwa 1:1, beträgt.

5. Formmassen nach einem oder mehreren der Ansprüche 1 bis 4, **dadurch gekennzeichnet,** daß das Polyepoxidharz ein epoxidierter Novolak mit einer Epoxidzahl von 0,3 bis 0,6 und einem Gesamtgehalt an hydrolysierbarem Halogen < 0,6 Gew.-%, vorzugsweise < 0,1 Gew.-%, ist.

6. Formmassen nach Anspruch 5, **dadurch gekennzeichnet,** daß das Polyepoxidharz ein Gemisch aus epoxidiertem Novolak und Triglycidylisocyanurat ist.

7. Formmassen nach Anspruch 5, **dadurch gekennzeichnet,** daß das Polyepoxidharz ein Gemisch aus epoxidiertem Novolak und einem kernhalogenierten Bisphenol-bisglycidylether und/oder einem kernbromierten epoxidierten Novolak ist, wobei der Gesamtgehalt des Gemisches an kerngebundenem Halogen ≤ 4 Gew.-% beträgt.

8. Formmassen nach einem oder mehreren der Ansprüche 1 bis 7, **dadurch gekennzeichnet,** daß der Füllstoff Quarzmehl, Quarzgutmehl oder künstlich hergestelltes Siliciumdioxid mit geringer α-Aktivität ist.

9. Formmassen nach einem oder mehreren der Ansprüche 1 bis 8, **dadurch gekennzeichnet,** daß der Füllstoffanteil 50 bis 85 Gew.-%, vorzugsweise 70 bis 80 Gew.-%, beträgt.

10. Formstoffe, hergestellt aus Formmassen nach einem oder mehreren der Ansprüche 1 bis 9.